# EUROPÄISCHE PATENTSCHRIFT

(11) **EP 0 617 732 B1**
(45) Veröffentlichungstag und Bekanntmachung des Hinweises auf die Patenterteilung: **06.09.1995**
(21) Anmeldenummer: 92924690.8
(22) Anmeldetag: 08.12.1992
(51) Int. Cl.: C11C 3/00, C07C 303/02, C14C 9/02, C11D 1/28, A61K 7/48, A61K 7/06

(54) **VERFAHREN ZUR HERSTELLUNG HYDROPHILISIERTER TRIGLYCERIDE**
METHOD OF PRODUCING TRIGLYCERIDES WITH HYDROPHILIC PROPERTIES
PROCEDE DE FABRICATION DE TRIGLYCERIDES HYDROPHILISES

(30) Priorität: 17.12.1991 DE 4141532
(43) Veröffentlichungstag der Anmeldung: 05.10.1994
(73) Patentinhaber: Henkel Kommanditgesellschaft auf Aktien, 40191 Düsseldorf (DE)
(72) Erfinder: WANGEMANN, Frank, D - 42697 Solingen 11 (DE); RATHS, Hans-Christian, D-40789 Monheim (DE); ZAUNS-HUBER, Rudolf, Dr., D-40589 Düsseldorf (DE)
(86) Internationale Anmeldenummer: EP9202835
(87) Internationale Veröffentlichungsnummer: WO9312215

(56) Entgegenhaltungen:
- EP-A- 0 247 509
- EP-A- 0 353 704
- WO-A-92/09570
- WO 91/06531

## Beschreibung

### Gebiet der Erfindung

Die Erfindung betrifft ein verfahren zur Herstellung hydrophilisierter Triglyceride, bei dem man gesättigte, ungesättigte und/oder geblasene Triglyceride ethoxyliert, sulfiert und neutralisiert sowie die Verwendung dieser Produkte in oberflächenaktiven Mitteln.

### Stand der Technik

Hydrophilisierte Triglyceride stellen bekannte oberflächenaktive Verbindungen dar, die durch Sulfierung und/oder Ethoxylierung von Glyceriden hergestellt werden können.

In der Europäischen Patentanmeldung **EP 0 305 925 A1** wird beispielsweise vorgeschlagen, epoxidierte Ricinolglyceride mit gasförmigem Schwefeltrioxid umzusetzen.

Gemäß der Deutschen Patentanmeldung **DE 39 41 365 A1** sowie der Internationalen Patentanmeldung **WO 91/06532** lassen sich oberflächenaktive Substanzen erhalten, indem man gesättigte oder ungesättigte Triglyceride mit gasförmigem Schwefeltrioxid sulfiert.

Aus der Europäischen Patentanmeldung **EP 0 247 509 A1** sind des weiteren Lederfettungsmittel bekannt, zu deren Herstellung man ungesättigte Triglyceride zunächst ethoxyliert und/oder epoxidiert und die resultierenden Zwischenprodukte danach mit Schwefelsäure sulfatiert.

In der Deutschen Patentanmeldung **DE 34 32 219 A1** werden Parfumölsolubilisatoren vorgeschlagen, die erhältlich sind, indem man epoxidierte Triglyceride mit Nucleophilen einer Ringöffnung unterwirft und die dabei resultierenden Polyole anschließend ethoxyliert; die Internationale Patentanmeldung **WO 91/06531** beschreibt schließlich die Sulfatierung dieser Produkte.

Insgesamt weisen die genannten Verfahren entscheidende Nachteile auf, die eine technische Realisierung der Produkte in Frage stellen:
- Triglyceride, bei denen die Hydrophilisierung entweder durch Sulfierung oder Ethoxylierung erfolgt, zeigen für viele Anwendungen eine nicht ausreichende Wasserlöslichkeit.
- Die Anlagerung von Schwefelsäure an die Doppelbindungen ungesättigter, gegebenenfalls ethoxylierter Triglyceride gelingt nur im Überschuß des Sulfatierungsmittels und ist durch den Anstieg der Viskosität mit hohen technischen Problemen verbunden. Die nachfolgende Neutralisation der sauren Produkte führt infolge des Überschusses an Schwefelsäure zwangsläufig zu einer unerwünscht hohen Salzbelastung.
- Epoxidierte Triglyceride sind nur mit hohem technischen Aufwand zugänglich und somit aus wirtschaftlicher Sicht als Rohstoffe für die Herstellung der hydrophilisierten Triglyceride unvorteilhaft.

Die Aufgabe der Erfindung bestand somit darin, ein neues Verfahren zur Herstellung hydrophilisierter Triglyceride zu entwickeln, das frei von den geschilderten Nachteilen ist.

### Beschreibung der Erfindung

Gegenstand der Erfindung ist ein Verfahren zur Herstellung hydrophilisierter Triglyceride, das sich dadurch auszeichnet, daß man gesättigte, ungesättigte und/oder geblasene Triglyceride der Formel (I), 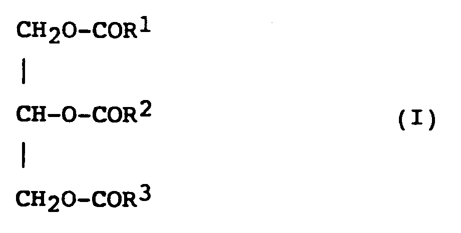
in der R¹CO, R²CO und R³CO unabhängig voneinander für gegebenenfalls hydroxysubstiutuierte Acylreste mit 6 bis 24 Kohlenstoffatomen und 0 oder 1 bis 5 Doppelbindungen stehen,
a) in Gegenwart von Glycerin und alkalischen Katalysatoren mit Ethylenoxid umsetzt,
b) die resultierenden ethoxylierten Triglyceride mit gasförmigem Schwefeltrioxid sulfiert und
c) die daraus resultierenden sauren Sulfierprodukte anschließend mit wäßrigen Basen neutralisiert.

Überraschenderweise wurde gefunden, daß sich ethoxylierte Triglyceride mit gasförmigem Schwefeltrioxid ohne Viskositätsprobleme gegebenenfalls auch kontinuierlich sulfieren lassen.

Unter **Triglyceriden** sind Vollester des Glycerins mit drei gleichen oder auch verschiedenen Fettsäuren zu verstehen. Vorzugsweise werden Triglyceride als Rohstoffe eingesetzt, die Iodzahlen im Bereich von 7 bis 180, insbesondere 80 bis 115 aufweisen.

Die Fettsäurekomponente der Triglyceride kann sich beispielsweise von folgenden Fettsäuren ableiten: Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Ricinolsäure, Arachinsäure, Gadoleinsäure, Behensäure, Erucasäure, Arachidonsäure und Clupanodonsäure. Bevorzugt werden Triglyceride der Formel **(I)** einsetzt, in der R¹CO, R²CO und R³CO unabhängig voneinander für Acylreste mit 16 bis 18 Kohlenstoffatomen und 0 oder 1 oder 2 Doppelbindungen stehen.

Die Triglyceride können synthetischer Herkunft sein; bevorzugt werden jedoch natürliche Einsatzstoffe, beispielsweise Palmöl, Palmkernöl, Kokosöl, Olivenöl, Rüböl alter und neuer Züchtung, Sonnenblumenöl alter und neuer Züchtung, Leinöl, Erdnußöl, Baumwollsaatöl, Korianderöl, Meadowfoamöl, Lardöl, Schweineschmalz, Rindertalg und Fischöl eingesetzt. Des weiteren kommen als Ausgangsprodukte auch sogenannte "geblasene Öle" in Betracht. Hierunter werden autoxidierte Triglyceride verstanden, die durch Einblasen von 100 bis 150°C heißer Luft in fette Öle erhalten werden [**Römpp Chemielexikon, Thieme Verlag, Stuttgart, 1990, S.1498**].

Die Triglyceride können rohstoffbedingt Anteile an Partialglyceriden enthalten, sofern diese jeweils 50 Gew.-% nicht übersteigen.

Die **Ethoxylierung** der Triglyceride kann nach an sich bekannten Verfahren durchgeführt werden. Als alkalische Katalysatoren kommen Alkalihydroxide, Erdalkalihydroxide und/oder Alkali-C₁-C₄-alkoholate in Betracht. Typische Beispiele hierfür sind Kaliumhydroxid, Bariumhydroxid, Kalium-tert.-butylat und insbesondere Natriumhydroxid und Natriummethylat. Die Ethoxylierung kann üblicherweise in Gegenwart von 0,1 bis 2 Gew.-% - bezogen auf Triglycerid - der alkalischen Katalysatoren durchgeführt werden. Zur Beschleunigung der Reaktion wird den Triglyceriden Glycerin in Mengen von 0,5 bis 5, vorzugsweise 1,5 bis 3,0 Gew.-% - bezogen auf Triglycerid - zugesetzt. Vorteilhafterweise wird die Ethoxylierung bei Temperaturen von 150 bis 180, insbesondere 160 bis 170°C und autogenen Drücken von 1 bis 5, insbesondere 2 bis 4 bar durchgeführt.

Das molare Verhältnis, in dem man die Triglyceride und das Ethylenoxid einsetzt, kann 1 : 0,8 bis 1 : 50, vorzugsweise 1 : 1 bis 1 : 10 betragen. Bei der Ethoxylierung findet eine Insertion des Ethylenoxids in die Carbonylesterbindungen statt, die statistischen Gesetzen folgt. Der aus dem molaren Verhältnis errechenbare Ethoxylierungsgrad stellt somit nur eine Durchschnittsgröße dar.

Die **Sulfierung** der ethoxylierten Triglyceride mit gasförmigem Schwefeltrioxid kann in der für Fettsäure-niedrigalkylester bekannten Weise [**J.Falbe (ed.), "Surfactants in consumer products", Springer Verlag, Berlin-Heidelberg, 1987, S. 61**] erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Die ethoxylierten Triglyceride und das Schwefeltrioxid können im molaren Verhältnis von 1 : 0,3 bis 1 : 3,0, vorzugsweise 1 : 0,9 bis 1 : 2,5 und insbesondere 1 : 1,0 bis 1 : 2 eingesetzt werden, wobei das Ziel auch darin bestehen kann, nicht alle, sondern nur einen Teil, beispielsweise 10 bis 25 %, der im Molekül vorhandenen Doppelbindungen zu sulfieren. Hierfür hat es sich als optimal erwiesen, die Sulfierung mit einem molaren Einsatzverhältnis von 1 : 1 bis 1 : 1,2 durchzuführen. Unter Berücksichtigung der Viskosität der Reaktionsprodukte kann die Sulfierung bei Temperaturen von 30 bis 90°C durchgeführt werden, wobei sich ein Bereich von 50 bis 80°C als optimal erwiesen hat.

Zur **Neutralisation** werden die bei der Sulfierung der ethoxylierten Triglyceride anfallenden sauren Sulfierprodukte in wäßrige Basen eingerührt und auf einen pH-Wert von 6,5 bis 8,5 eingestellt. Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine in Betracht. Die Neutralisationsbasen gelangen dabei vorzugsweise in Form 5 bis 55 gew.-%iger wäßriger Lösungen zum Einsatz, wobei 5 bis 25 gew.-%ige wäßrige Natriumhydroxidlösungen bevorzugt sind.

Zur Verbesserung der pH- und Lagerstabilität der Produkte empfiehlt es sich, die Neutralisation mit einem Überschuß von 10 bis 20 mol-% der Base - bezogen auf saures Reaktionsprodukt - bei Temperaturen von 70 bis 90°C durchzuführen. Zur Verbesserung des Sulfiergrades hat es sich ferner als vorteilhaft erwiesen, das saure Reaktionsprodukt nach dem Verlassen des Reaktors und vor der Neutralisation bei einer Temperatur von 70 bis 90°C über einen Zeitraum 0,1 bis 1 h einer Nachreaktion zu unterwerfen. Diese kann beispielsweise diskontinuierlich in einem Kessel oder kontinuierlich in einer Rohrschlange durchgeführt werden.

Die hydrophilisierten Triglyceride stellen komplexe Gemische dar, die neben ethoxylierten Mono-, Di- und Triglyceridsulfonaten mit innenständiger Sulfonsäuregruppierung unter anderem auch sulfonierte Fettsäureethoxylate, Glyceridethersulfate, Glycerinethersulfate, Glycerin und Seifen enthalten.

Die Reaktionsprodukte können nach Neutralisation in an sich bekannter Weise in Gegenwart von Wasserstoffperoxid- oder Natriumhypochloritlösung, gegebenenfalls unter Zusatz von Bleichboostern wie beispielsweise Magnesiumionen, gebleicht werden. Dabei werden, bezogen auf den Feststoffgehalt in der Lösung der Sulfierprodukte, 0,2 bis 2 Gew.-% Wasserstoffperoxid, berechnet als 100 %ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

Die nach dem erfindungsgemäßen Verfahren erhältlichen hydrophilisierten Triglyceride weisen oberflächenaktive Eigenschaften auf, sind gut wasserlöslich, fördern die Emulgierung ansonsten nicht miteinander msichbarer Stoffe und können leicht konfektioniert werden.

Ein weiterer Gegenstand der Erfindung betrifft daher die Verwendung der Produkte zur Herstellung von Wasch-, Spül- und Reinigungsmitteln, Lederfettungsmittel sowie Produkten der Haar und Körperpflege, in denen sie in Mengen von 0,1 bis 25, vorzugsweise 1 bis 10 Gew.-% - bezogen auf die Mittel - enthalten sein können.

### Beispiele

### Beispiele 1 bis 4:

I. Ethoxylierung:
   In einem 2-l-Stahlautoklaven wurde eine Mischung aus 889 g (1,0 mol) Rüböl neuer Züchtung (Ölsäureanteil > 80 Gew.-%, Iodzahl 108), 20 g (0,2 mol) Glycerin und 4 g Natriummethylat in Form einer 30 gew.-%igen Lösung in Methanol vorgelegt. Das Druckgefäß wurde dreimal abwechselnd evakuiert und mit Stickstoff beaufschlagt, verschlossen und auf eine Temperatur von 175°C erhitzt. Anschließend wurden über einen Heber 44 bis 220 g (1 bis 5 mol) Ethylenoxid über einen Zeitraum von 0,5 bis 2 h portionsweise eindosiert, wobei sich ein autogener Druck von 4 bar einstellte. Nach Beendigung der Zugabe ließ man weitere 0,5 h nachreagieren, danach wurde der Autoklav abekühlt und entspannt. Das ethoxylierte Rüböl wurde in praktisch quantitativer Ausbeute als leicht gelb gefärbte, niedrigviskose Flüssigkeit erhalten.
II. Sulfierung:
   In einem kontinuierlich arbeitenden Fallfilmreaktor (Länge 120 cm, Querschnitt 1 cm, Eduktdurchsatz 600 g/h) mit Mantelkühlung und seitlicher SO₃-Begasung wurden jeweils 5 mol der Ethoxylate A1 bis A4 bei einer Temperatur T¹ von 75°C mit 480 g (6,0 mol) gasförmigem Schwefeltrioxid (5 Vol.-% in Luft) - entsprechend einem molaren Einsatzverhältnis Ethoxylat : SO₃ = 1 : 1,2 - umgesetzt. Die sauren Sulfierprodukte wurden kontinuierlich bei einer Temperatur T² von 80°C in eine 37 gew.- %ige wäßrige Natriumhydroxidlösung eingetragen und bei pH = 7,5 neutralisiert. Die Produkte wurden in Form leicht beweglicher opaker Flüssigkeiten erhalten. Die Ergebnisse sind in Tab.1 zusammengefaßt.

**Tab.1**

| Hydrophilisierte Triglyceride Prozentangaben als Gew.-% | | | | | | | |
|---|---|---|---|---|---|---|---|
| Bsp. | Ethoxylierung | | | Sulfierung | | | |
| | Eth. | EO | t(Eth) h | WAS mEq/g | US % | SO₄²⁻ % | H₂O % |
| 1 | A1 | 1 | 0,5 | 0,034 | 35,6 | 0,7 | 47,7 |
| 2 | A2 | 2 | 1,0 | 0,030 | 33,3 | 0,7 | 50,3 |
| 3 | A3 | 3 | 1,5 | 0,027 | 31,4 | 0,5 | 50,8 |
| 4 | A4 | 5 | 2,0 | 0,023 | 28,3 | 0,6 | 51,5 |
| Legende: Eth. = Ethylenoxidaddukt EO = durchschnittlicher Ethoxylierungsgrad t(Eth) = Ethoxylierungszeit | | | | | | | |

Der Gehalt an Waschaktiver Substanz (WAS) und die unsulfierten Anteile (US) wurden nach den DGF-Einheitsmethoden, Stuttgart, 1950-1984, H-III-10 bzw. G-II-6b ermittelt. Der Sulfatgehalt wurde als Natriumsulfat berechnet, die Bestimmung des Wassergehaltes erfolgte nach der Fischer-Methode.

## Patentansprüche

1. Verfahren zur Herstellung hydrophilisierter Triglyceride, **dadurch gekennzeichnet**, daß man gesättigte, ungesättigte und/oder geblasene Triglyceride der Formel (I), 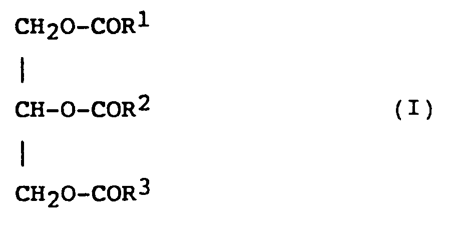 in der R¹CO, R²CO und R³CO unabhängig voneinander für gegebenenfalls hydroxysubstituierte Acylreste mit 6 bis 24 Kohlenstoffatomen und 0 oder 1 bis 5 Doppelbindungen stehen,
a) in Gegenwart von Glycerin und alkalischen Katalysatoren mit Ethylenoxid umsetzt,
b) die resultierenden ethoxylierten Triglyceride mit gasförmigem Schwefeltrioxid sulfiert und
c) die daraus resultierenden sauren Sulfierprodukte anschließend mit wäßrigen Basen neutralisiert.

2. Verfahren nach Anspruch 1, **dadurch gekennzeichnet**, daß man Triglyceride der Formel **(I)** einsetzt, in der R¹CO, R²CO und R³CO unabhängig voneinander für Acylreste mit 16 bis 18 Kohlenstoffatomen und 0, 1 oder 2 Doppelbindungen stehen.

3. Verfahren nach den Ansprüchen 1 und 2, **dadurch gekennzeichnet**, daß man ungesättigte Triglyceride einsetzt, die Iodzahlen von 7 bis 180 aufweisen.

4. Verfahren nach mindestens einem der Ansprüche 1 bis 3, **dadurch gekennzeichnet**, daß man als alkalische Katalysatoren für die Ethoxylierung Alkylihydroxide, Erdalkalihydroxide und/oder Alkali-C₁-C₄-alkoholate einsetzt.

5. Verfahren nach mindestens einem der Ansprüche 1 bis 4, **dadurch gekennzeichnet**, daß man die Ethoxylierung in Gegenwart von 0,1 bis 2 Gew.-% - bezogen auf Triglycerid - der alkalischen Katalysatoren durchführt.

6. Verfahren nach mindestens einem der Ansprüche 1 bis 5, **dadurch gekennzeichnet**, daß man die Ethoxylierung in Gegenwart von 0,5 bis 5 Gew.-% - bezogen auf Triglycerid - Glycerin durchführt.

7. Verfahren nach mindestens einem der Ansprüche 1 bis 6, **dadurch gekennzeichnet**, daß man die ungesättigten Triglyceride und das Ethylenoxid im molaren Verhältnis von 1 : 0,8 bis 1 : 50 einsetzt.

8. Verfahren nach mindestens einem der Ansprüche 1 bis 7, **dadurch gekennzeichnet**, daß man die Ethoxylierung bei Temperaturen von 150 bis 180°C und autogenen Drücken von 1 bis 5 bar durchführt.

9. Verfahren nach mindestens einem der Ansprüche 1 bis 8, **dadurch gekennzeichnet**, daß man die ethoxylierten Triglyceride und das Schwefeltrioxid im molaren Verhältnis von 1 : 0,3 bis 1 : 3,0 einsetzt.

10. Verfahren nach mindestens einem der Ansprüche 1 bis 9, **dadurch gekennzeichnet**, daß man die Sulfierung bei Temperaturen von 30 bis 90°C durchführt.

11. Verfahren nach mindestens einem der Ansprüche 1 bis 10, **dadurch gekennzeichnet**, daß man die Sulfierung kontinuierlich in einem Reaktor durchführt, der nach dem Fallfilmprinzip arbeitet.

12. Verfahren nach mindestens einem der Ansprüche 1 bis 11, **dadurch gekennzeichnet**, daß man die Neutralisation mit 5 bis 55 gew.-%igen wäßrigen Basen aus der von Alkalimetallhydroxiden, Erdalkalimetalloxiden und -hydroxiden, Ammoniak, Mono-, Di- und Tri-C₂₋₄-Alkanolaminen sowie primären, sekundären und tertiären C₁₋₄-Alkylaminen gebildeten Gruppe durchführt.

13. Verfahren nach mindestens einem der Ansprüche 1 bis 12, **dadurch gekennzeichnet**, daß man den pH-Wert während der Neutralisation in einem Bereich von 6,5 bis 8,5 hält.

14. Verwendung der hydrophilisierten Triglyceride nach dem Verfahren nach den Ansprüchen 1 bis 13 in Wasch-, Spül- und Reinigungsmitteln sowie Produkten zur Haar- und Körperpflege.

## Claims

1. A process for the production of hydrophilicized triglycerides, characterized in that saturated, unsaturated and/or blown triglycerides corresponding to formula (I): 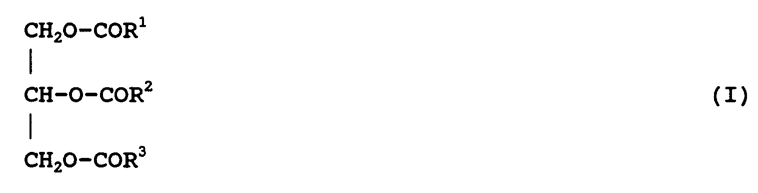 in which R¹CO, R²CO and R³CO independently of one another represent optionally hydroxy-substituted acyl radicals containing 6 to 24 carbon atoms and 0 or 1 to 5 double bonds,
a) are reacted with ethylene oxide in the presence of glycerol and alkaline catalysts,
b) the resulting ethoxylated triglycerides are sulfonated with gaseous sulfur trioxide and
c) the resulting acidic sulfonation products are subsequently neutralized with aqueous bases.

2. A process as claimed in claim 1, characterized in that triglycerides corresponding to formula (I), in which R¹CO, R²CO and R³CO independently of one another represent acyl radicals containing 16 to 18 carbon atoms and 0, 1 or 2 double bonds, are used.

3. A process as claimed in claims 1 and 2, characterized in that unsaturated triglycerides with iodine values of 7 to 180 are used.

4. A process as claimed in at least one of claims 1 to 3, characterized in that alkali metal hydroxides, alkaline earth metal hydroxides and/or alkali metal C₁₋₄ alcoholates are used as the alkaline ethoxylation catalysts.

5. A process as claimed in at least one of claims 1 to 4, characterized in that the ethoxylation is carried out in the presence of 0.1 to 2% by weight, based on triglyceride, of the alkaline catalysts.

6. A process as claimed in at least one of claims 1 to 5, characterized in that the ethoxylation is carried out in the presence of 0.5 to 5% by weight, based on triglyceride, of glycerol.

7. A process as claimed in at least one of claims 1 to 6, characterized in that the unsaturated triglycerides and the ethylene oxide are used in a molar ratio of 1:0.8 to 1:50.

8. A process as claimed in at least one of claims 1 to 7, characterized in that the ethoxylation is carried out at temperatures of 150 to 180°C under autogenous pressures of 1 to 5 bar.

9. A process as claimed in at least one of claims 1 to 8, characterized in that the ethoxylated triglycerides and the sulfur trioxide are used in a molar ratio of 1:0.3 to 1:3.0.

10. A process as claimed in at least one of claims 1 to 9, characterized in that the sulfonation is carried out at temperatures of 30 to 90°C.

11. A process as claimed in at least one of claims 1 to 10, characterized in that the sulfonation is carried out continuously in a reactor operating on the falling film principle.

12. A process as claimed in at least one of claims 1 to 11, characterized in that the neutralization is carried out with 5 to 55% by weight aqueous bases from the group consisting of alkali metal hydroxides, alkaline earth metal oxides and hydroxides, ammonia, mono-, di- and tri-C₂₋₄-alkanolamines and also primary, secondary and tertiary C₁₋₄ alkylamines.

13. A process as claimed in at least one of claims 1 to 12, characterized in that the pH value is kept at 6.5 to 8.5 during the neutralization step.

14. The use of the hydrophilicized triglycerides produced by the process claimed in claims 1 to 13 in laundry detergents, dishwashing detergents and cleaning products and also in hair-care and personal hygiene products.

## Revendications

1. Procédé de préparation de triglycérides hydrophilisés, caractérisé par des triglycérides saturés, insaturés et/ou soufflés de formule (I) 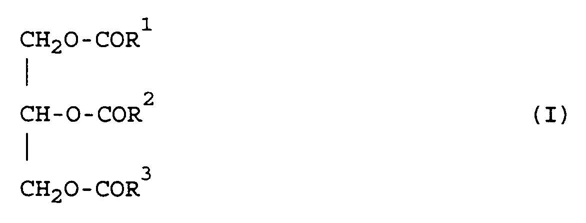 dans laquelle R¹CO, R²CO et R³CO indépendamment l'un de l'autre représentent des radicaux acyle hydroxy substitués de 6 à 24 atomes de carbone avec 0 ou 1 à 5 double liaisons, en ce que
a) on les fait réagir en présence de glycérine et de catalyseurs alcalins avec de l'oxyde d'éthylène,
b) on sulfone les triglycérides obtenus avec du trioxyde de soufre gazeux et,
c) on neutralise ensuite les produits de sulfonation acides obtenus avec des bases aqueuses.

2. Procédé selon la revendication 1, caractérisé en ce qu'on utilise des triglycérides de formule (I), dans laquelle R¹CO, R²CO et R³Co représentent indépendamment l'un de l'autre des radicaux acyle de 16 à 18 atomes de C et 0 ou 1 ou 2 doubles liaisons.

3. Procédé selon les revendications 1 et 2, caractérisé en ce qu'on utilise des triglycérides insaturés, qui ont des indices d'iode de 7 à 180.

4. Procédé selon au moins une des revendications 1 à 3, caractérisé en ce qu'on utilise comme catalyseurs alcalins pour l'éthoxylation des hydroxydes alcalins, des hydroxydes alcalino-terreux et/ou des alcoolats en C₁-C₄ d'alcalins.

5. Procédé selon au moins une des revendications 1 à 4, caractérisé en ce qu'on réalise l'éthoxylation en présence de 0,1 à 2 % en poids de catalyseurs alcalins, par rapport aux triglycérides.

6. Procédé selon au moins l'une des revendications 1 à 5, caractérisé en ce qu'on réalise l'éthoxylation en présence de 0,5 à 5 % en poids de glycérol, par rapport aux triglycérides.

7. Procédé selon au moins l'une des revendications 1 à 6, caractérisé en ce qu'on utilise les triglycérides insaturés et l'oxyde d'éthylène en proportion molaire de 1:0,8 à 1:50.

8. Procédé selon au moins l'une des revendications 1 à 7, caractérisé en ce qu'on réalise l'éthoxylation à des températures de 150 à 180°C sous des pressions autogènes de 1 à 5 bars.

9. Procédé selon au moins l'une des revendications 1 à 8, caractérisé en ce qu'on utilise les triglycérides éthoxylés et le trioxyde de soufre en proportion molaire de 1:0,3 à 1:3,0.

10. Procédé selon au moins l'une des revendications 1 à 9, caractérisé en ce qu'on réalise la sulfonation à des températures de 30 à 90°C.

11. Procédé selon au moins l'une des revendications 1 à 10, caractérisé en ce qu'on réalise la sulfonation en continu dans un réacteur, qui fonctionne selon le principe du ruissellement.

12. Procédé selon au moins l'une des revendications 1 à 11, caractérisé en ce qu'on réalise la neutralisation avec des bases aqueuses de 5 à 55 % en poids, choisies dans le groupe formé des hydroxydes de métaux alcalins, des oxydes et hydroxydes de métaux alcalinoterreux, de l'ammoniaque, des mono-, di- et tri-(alcanol en C₂₋₄)-amines ainsi que des (alkyl en C₁₋₄ ) amines primaires, secondaires et tertiaires.

13. Procédé selon au moins l'une des revendications 1 à 12, caractérisé en ce qu'on maintient le pH pendant la neutralisation dans un intervalle de 6,5 à 8,5.

14. Utilisation de triglycérides hydrophilisés selon le procédé, d'après les revendications 1 à 13, dans des agents de lavage, de rinçage et de nettoyage ainsi que dans des produits pour les soins du corps et des cheveux.
